# EUROPEAN PATENT APPLICATION

(11) **EP 4 001 309 A1**
(43) Date of publication of application: **25.05.2022**
(21) Application number: 20840949.0
(22) Date of filing: 16.07.2020
(51) Int. Cl.: C07K 16/28, C07K 16/46, C12N 15/13, A61K 39/395, A61P 35/00

(54) **ANTI-CD47/ANTI-CTLA-4 BISPECIFIC ANTIBODY AND PREPARATION METHOD THEREFOR AND APPLICATION THEREOF**

(30) Priority: 16.07.2019 CN 201910640002
(71) Applicant: Nanjing GenScript Biotech Co., Ltd., Nanjing, Jiangsu 211100 (CN)
(72) Inventor: YIN, Liusong, Nanjing, Jiangsu 211100 (CN); LI, Zhongdao, Nanjing, Jiangsu 211100 (CN); ZHOU, Tielin, Nanjing, Jiangsu 211100 (CN); FANG, Zhuo, Nanjing, Jiangsu 211100 (CN)
(74) Representative: Ström & Gulliksson AB
(86) International application number: PCT/CN2020/102324
(87) International publication number: WO 2021/008577

(57) **Abstract**

The present invention relates to an anti-CD47/anti-CTLA-4 bispecific antibody and a preparation method thereof and an application thereof. The bispecific antibody comprises (a) a first antigen-binding portion, comprising a heavy chain variable region (V_{H}) and a light chain variable region (V_{L}), where V_{H} and V_{L} form an antigen-binding site that specifically binds to CD47; and (b) a second antigen-binding portion comprising a single domain antibody (sdAb) that specifically binds to CTLA-4, the first antigen-binding portion and the second antigen-binding portion are fused to each other. The bispecific antibody to which the present invention relates may simultaneously block two means of tumor immune escape, and therefore has a better effect in tumor immunotherapy.

## Description

### TECHNICAL FIELD

The present invention relates to the field of antibodies, and specifically, to a bispecific antibody, a preparation method thereof, and use thereof. The bispecific antibody includes a first antigen binding portion that specifically binds to CD47 and a second antigen binding portion that specifically binds to CTLA-4.

### BACKGROUND

The mammalian immune system is a host defense system that protects a mammal from microbial infections and cancers (Chen et al., Frontiers Immunol. 9:320 (2018)). The immune system all over the body is an extremely complex network system. Different immune cells and specific tissues and organs exert a synergistic effect to form the immune system. When the immune system is functioning normally, diseased cells in the host body will be recognized from healthy cells and eliminated, thereby ensuring the stability of the body's environment. Therefore, maintaining the integrity of the immune system is essential to maintaining our own health. Conversely, loss of control of the immune system will cause autoimmune diseases, inflammation, cancers, etc (Ribas et al., Cancer Discovery 5:915-9 (2015); Yao and Chen, Eur. J. Immunol. 43:576-9 (2013)). The immune system can be divided into two categories, namely humoral immunity and cell-mediated immunity. Antibodies and other biological macromolecules regulate humoral immunity. In contrast, cell-mediated immunity is regulated at the cellular level, involving the activation of macrophages, natural killer cells, and antigen-specific killer T cells.

Activation and suppression of immune response are mainly regulated by two independent signaling pathways (Gorentla and Zhong, J. Clin. Cell. Immunol. (2012); Huse, J. Cell Sci. 122:1269-73 (2009); Mizota et al., J. Anesthesia 27:80-7 (2013)). The first signal is antigen-mediated. The first signal is generated when the T-cell receptor specifically recognizes and binds to the antigen peptide presented by the MHC on the surface of the antigen-presenting cell (APC). The second signal is provided by the interaction between the APCs and costimulators expressed on the surface of T cells. T cells can kill tumors only when the first and second signals are activated in turn. If the second signal is lacking, T cells will enter a state of unresponsiveness or will be immune tolerance, and even cause programmed cell death. As described above, the second signaling pathway is very important to the activation of immune cells. Specifically, co-stimulatory and co-inhibitory receptors participate in the second signaling pathway to perform immune response and regulation on antigen-receptor presentation and balance positive and negative signals while maintaining immune tolerance to autoantigens, maximizing the immune response to invaders (Chen and Flies, Nat. Rev. Immunol. 13:227-42 (2013); Ewing et al., Int. J. Cardiol. 168:1965-74 (2013); Liu et al., Immunol. Invest. 45:813-31 (2016); Shen et al., Frontiers in Biosci. 24:96-132 (2019); Zhang and Vignali, Immunity 44:1034-51 (2016)).

CD28 is a member of the CD28 family and is a major T-cell co-stimulatory receptor, constitutively expressed on initial CD4+ and CD8+ T cells. The cytotoxic T lymphocyte-associated antigen 4 (CTLA-4) molecule is also a member of the CD28 receptor family, but it is a co-inhibitory receptor, this molecule is constitutively expressed on regulatory T cells (Tregs) or produced after activation of T cells by CD28. Both CD28 and CTLA-4 can bind to the B7.1 (CD80) or B7.2 (CD86) ligand to transmit activation or suppression signals in T cells. Therefore, blocking the immune checkpoint B7/CTLA-4 can prevent the generation of T cell suppression signals and enhance the specific anti-tumor immune response. At present, the only CTLA-4 antibody approved for marketing is Ipilimumab (the brand name Yervoy) of BMS, and it is used for melanoma immunotherapy. However, in view of clinical adverse effects of the Ipilimumab antibody, the immunotherapy against the CTLA-4 target needs to be further optimized.

CD47, also known as integrin associated protein, is a transmembrane protein that is encoded by the CD47 gene, and belongs to the immunoglobulin superfamily. CD47 is widely expressed on the surface of normal cells and can interact with signal-regulatory protein alpha (SIRPα), thrombospondin-1 (TSP-1), and integrin to mediate cell apoptosis, proliferation, and immunity responses, and the like. CD47, as an innate immune checkpoint receptor, binds to SIRPα mainly expressed on macrophages and dendritic cells and then releases a "don't eat me" signal to the macrophages to inhibit phagocytosis, thereby avoiding the attack of the body's immune system. Cancer cells escape phagocytosis by upregulating the expression of CD47, thereby evading immune surveillance. The overexpression of CD47 in blood and solid tumors is highly correlated with the poor prognosis of clinical treatment. Therefore, the use of anti-CD47 antibodies or high-affinity SIRPα variants to block the CD47-SIRPα signaling pathway has become a potential strategy to promote the phagocytosis of tumor cells by macrophages. However, in view of the wide expression of CD47, the anti-CD47 antibodies have a high risk of binding to healthy cells, especially red blood cells, which will increase the risk of blood toxicity. In addition, more and more studies have shown that blocking CD47 alone is not sufficient to generate anti-tumor immunity in immunocompetent hosts. Moreover, researchers at Stanford University reported that the interference in the CD47/SIRPα pathway by SIRPα treatment cannot induce phagocytosis (Sockolosky et al., PNAS 113:E2646-2654 (2016)). Therefore, considering the effectiveness and safety of cancer treatment, the anti-CD47 antibodies need to be further optimized to improve tumor targeting specificity.

### SUMMARY

According to an aspect, the present invention provides an isolated bispecific binding protein, and the protein includes a first antigen binding portion that specifically binds to CD47 and a second antigen binding portion that specifically binds to CTLA-4. Specifically, the present invention provides an isolated anti-CD47/anti-CTLA-4 bispecific antigen-binding protein or a fragment thereof, including (a) a first antigen binding portion including a heavy chain variable region (V_{H}) and a light chain variable region (V_{L}), V_{H} and V_{L} forming an antigen binding site that specifically binds to CD47; and (b) a second antigen binding portion including a single-domain antibody (sdAb) that specifically binds to CTLA-4, where the first antigen binding portion and the second antigen binding portion are fused to each other.

In some embodiments, the V_{H} of the first antigen binding portion includes heavy chain complementarity-determining regions (CDRs) HCDR1, HCDR2, and HCDR3, and the amino acid sequences of the HCDR1, HCDR2, and HCDR3 are respectively as set forth in SEQ ID NO:21, SEQ ID NO:22, and SEQ ID NO:23, or the sequences respectively including at most three (three, two, or one) amino acid mutations thereto; and the V_{L} of the first antigen binding portion includes light chain CDRs LCDR1, LCDR2, and LCDR3, the amino acid sequences of the LCDR1, LCDR2, and LCDR3 are respectively as set forth in SEQ ID NO:24, SEQ ID NO:25, and SEQ ID NO:26, or the sequences respectively including at most three (three, two, or one) amino acid mutations thereto. In some embodiments, the V_{H} of the first antigen binding portion includes heavy chain CDRs HCDR1, HCDR2, and HCDR3, the amino acid sequences of the HCDR1, HCDR2, and HCDR3 are respectively as set forth in SEQ ID NO:21, SEQ ID NO:22, and SEQ ID NO:23, or the sequences respectively including at most three (three, two, or one) amino acid substitutions thereto; and the V_{L} of the first antigen binding portion includes light chain CDRs LCDR1, LCDR2, and LCDR3, the amino acid sequences of the LCDR1, LCDR2, and LCDR3 are respectively as set forth in SEQ ID NO:24, SEQ ID NO:25, and SEQ ID NO:26, or the sequences respectively including at most three (three, two, or one) amino acid substitutions thereto. In some embodiments, the V_{H} of the first antigen binding portion includes heavy chain CDRs HCDR1, HCDR2, and HCDR3, and the amino acid sequences of the HCDR1, HCDR2, and HCDR3 are respectively as set forth in SEQ ID NO:21, SEQ ID NO:22, and SEQ ID NO:23; and the V_{L} of the first antigen binding portion includes light chain CDRs LCDR1, LCDR2, and LCDR3, and the amino acid sequences of the LCDR1, LCDR2, and LCDR3 are respectively as set forth in SEQ ID NO:24, SEQ ID NO:25, and SEQ ID NO:26.

In some embodiments, sdAb of the second antigen binding portion includes CDRs CDR1, CDR2, and CDR3, the amino acid sequences of the CDRs CDR1, CDR2, and CDR3 are respectively as set forth in SEQ ID NO:29, SEQ ID NO:30, and SEQ ID NO:31, or the sequences respectively including at most three (three, two, or one) amino acid mutations thereto. In some embodiments, sdAb of the second antigen binding portion includes CDRs CDR1, CDR2, and CDR3, the amino acid sequences of the CDRs CDR1, CDR2, and CDR3 are respectively as set forth in SEQ ID NO:29, SEQ ID NO:30, and SEQ ID NO:31, or the sequences respectively including at most three (three, two, or one) amino acid substitutions thereto. In some specific embodiments, sdAb of the second antigen binding portion includes CDRs CDR1, CDR2, and CDR3, and the amino acid sequences of the CDRs CDR1, CDR2, and CDR3 are respectively as set forth in SEQ ID NO:29, SEQ ID NO:30, and SEQ ID NO:31.

In some embodiments, the first antigen binding portion is a full-length antibody including two heavy chains and two light chains, the heavy chain includes V_{H}, and the light chain includes V_{L}.

In some embodiments, the first antigen binding portion and the second antigen binding portion are fused. In some embodiments, the C-terminus of the second antigen binding portion is fused to the N-terminus of at least one heavy chain of the first antigen binding portion or the N-terminus of at least one light chain of the first antigen binding portion. In some embodiments, the N-terminus of the second antigen binding portion is fused to the C-terminus of at least one heavy chain of the first antigen binding portion or the C-terminus of at least one light chain of the first antigen binding portion.

In some embodiments, the first antigen binding portion and the second antigen binding portion are fused by a peptide bond or a peptide linker. In some embodiments, the peptide linker is selected from a mutated human IgG1 hinge region or a GS linker. In some preferred embodiments, the amino acid sequence of the peptide linker is as set forth in SEQ ID NO:33, SEQ ID NO:35, or SEQ ID NO:37.

In some embodiments, the heavy chain of the first antigen binding portion includes a sequence that is at least 80%, 85%, 90%, 95%, 96%, 97%, 98%, or 99% identical to the amino acid sequence as set forth in SEQ ID NO:4, and the light chain of the first antigen binding portion includes a sequence that is at least 80%, 85%, 90%, 95%, 96%, 97%, 98%, or 99% identical to the amino acid sequence as set forth in SEQ ID NO:6. In some embodiments, the heavy chain of the first antigen binding portion includes a sequence that is at least 95% identical to the amino acid sequence as set forth in SEQ ID NO:4, and the light chain of the first antigen binding portion includes a sequence that is at least 95% identical to the amino acid sequence as set forth in SEQ ID NO:6. In some specific embodiments, the heavy chain of the first antigen binding portion includes the amino acid sequence as set forth in SEQ ID NO:4, and the light chain of the first antigen binding portion includes the amino acid sequence as set forth in SEQ ID NO:6.

In some embodiments, the second antigen binding portion includes a sequence that is at least 80%, 85%, 90%, 95%, 96%, 97%, 98%, or 99% identical to the amino acid sequence as set forth in SEQ ID NO:28. In some embodiments, the second antigen binding portion includes a sequence that is at least 95% identical to the amino acid sequence as set forth in SEQ ID NO:28. In some specific embodiments, the second antigen binding portion includes the amino acid sequence as set forth in SEQ ID NO:28.

In some embodiments, an isolated anti-CD47/anti-CTLA-4 bispecific antigen-binding protein or a fragment thereof is provided, the heavy chain of the first antigen binding portion includes a sequence that is at least 80%, 85%, 90%, 95%, 96%, 97%, 98%, or 99% identical to the amino acid sequence as set forth in SEQ ID NO:4, and the light chain of the first antigen binding portion includes a sequence that is at least 80%, 85%, 90%, 95%, 96%, 97%, 98%, or 99% identical to the amino acid sequence as set forth in SEQ ID NO:6; and the second antigen binding portion includes a sequence that is at least 80%, 85%, 90%, 95%, 96%, 97%, 98%, or 99% identical to the amino acid sequence as set forth in SEQ ID NO:28. In some embodiments, an isolated anti-CD47/anti-CTLA-4 bispecific antigen-binding protein or a fragment thereof is provided; the heavy chain of the first antigen binding portion includes a sequence that is at least 95% identical to the amino acid sequence as set forth in SEQ ID NO:4, and the light chain of the first antigen binding portion includes a sequence that is at least 95% identical to the amino acid sequence as set forth in SEQ ID NO:6; and the second antigen binding portion includes a sequence that is at least 95% identical to the amino acid sequence as set forth in SEQ ID NO:28. In some embodiments, an isolated anti-CD47/anti-CTLA-4 bispecific antigen-binding protein or a fragment thereof is provided; the heavy chain of the first antigen binding portion includes the amino acid sequence as set forth in SEQ ID NO:4, and the light chain of the first antigen binding portion includes the amino acid sequence as set forth in SEQ ID NO:6; and the second antigen binding portion includes the amino acid sequence as set forth in SEQ ID NO:28.

In some embodiments, the first antigen binding portion includes a human, humanized, or chimeric antibody or a fragment thereof. In some embodiments, the second antigen binding portion includes sdAb that specifically binds to CTLA-4, and the sdAb is a camelid, chimeric, humanized, or human antibody.

In some embodiments, an isolated anti-CD47/anti-CTLA-4 bispecific antigen-binding protein or a fragment thereof is provided, including an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide includes a sequence that is at least 80%, 85%, 90%, 95%, 96%, 97%, 98%, or 99% identical to the amino acid sequence as set forth in SEQ ID NO:8 or SEQ ID NO: 12, and the light chain polypeptide includes a sequence that is at least 80%, 85%, 90%, 95%, 96%, 97%, 98%, or 99% identical to the amino acid sequence as set forth in SEQ ID NO:6. In some embodiments, an isolated anti-CD47/anti-CTLA-4 bispecific antigen-binding protein or a fragment thereof is provided, including an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide includes a sequence that is at least 95% identical to the amino acid sequence as set forth in SEQ ID NO:8 or SEQ ID NO:12, and the light chain polypeptide includes a sequence that is at least 95% identical to the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide includes the amino acid sequence as set forth in SEQ ID NO:8 or SEQ ID NO: 12, and the light chain polypeptide includes the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide includes the amino acid sequence as set forth in SEQ ID NO:8, and the light chain polypeptide includes the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide includes the amino acid sequence as set forth in SEQ ID NO: 12, and the light chain polypeptide includes the amino acid sequence as set forth in SEQ ID NO:6.

In some embodiments, another isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide includes a sequence that is at least 80%, 85%, 90%, 95%, 96%, 97%, 98%, or 99% identical to the amino acid sequence as set forth in SEQ ID NO: 10 or SEQ ID NO: 14, and the light chain polypeptide includes a sequence that is at least 80%, 85%, 90%, 95%, 96%, 97%, 98%, or 99% identical to the amino acid sequence as set forth in SEQ ID NO:6. In some embodiments, another isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide includes a sequence that is at least 95% identical to the amino acid sequence as set forth in SEQ ID NO:10 or SEQ ID NO:14, and the light chain polypeptide includes a sequence that is at least 95% identical to the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide includes the amino acid sequence as set forth in SEQ ID NO: 10 or SEQ ID NO: 14, and the light chain polypeptide includes the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide includes the amino acid sequence as set forth in SEQ ID NO: 10, and the light chain polypeptide includes the amino acid sequence as set forth in SEQ ID NO:6. In some specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide includes the amino acid sequence as set forth in SEQ ID NO:14, and the light chain polypeptide includes the amino acid sequence as set forth in SEQ ID NO:6.

In some embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide includes a sequence that is at least 80%, 85%, 90%, 95%, 96%, 97%, 98%, or 99% identical to the amino acid sequence as set forth in SEQ ID NO: 16 or SEQ ID NO: 18, and the heavy chain polypeptide includes a sequence that is at least 80%, 85%, 90%, 95%, 96%, 97%, 98%, or 99% identical to the amino acid sequence as set forth in SEQ ID NO:4. In some embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide includes a sequence that is at least 95% identical to the amino acid sequence as set forth in SEQ ID NO:16 or SEQ ID NO: 18, and the heavy chain polypeptide includes a sequence that is at least 95% identical to the amino acid sequence as set forth in SEQ ID NO:4. In some specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide includes the amino acid sequence as set forth in SEQ ID NO: 16 or SEQ ID NO: 18, and the heavy chain polypeptide includes the amino acid sequence as set forth in SEQ ID NO:4. In some other specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide includes the amino acid sequence as set forth in SEQ ID NO: 16, and the heavy chain polypeptide includes the amino acid sequence as set forth in SEQ ID NO:4. In some specific embodiments, an isolated anti-CD47/anti-CTLA-4 bispecific antigen-binding protein or a fragment thereof is provided, including an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide includes the amino acid sequence as set forth in SEQ ID NO: 18, and the heavy chain polypeptide includes the amino acid sequence as set forth in SEQ ID NO:4.

According to another aspect, the present invention provides an isolated polynucleotide encoding the anti-CD47/anti-CTLA-4 bispecific antigen-binding protein or the fragment thereof. It can be commonly known to those skilled in the art that the change (such as replacement or deletion) of sequences encoding the protein does not change the amino acid of the protein. In some embodiments, the polynucleotide encoding the heavy chain fusion protein of the anti-CD47/anti-CTLA-4 bispecific antigen-binding protein or the fragment thereof includes a sequence that is at least 80%, 85%, 90%, 95%, 96%, 97%, 98%, or 99% identical to the nucleotide sequence as set forth in SEQ ID NO:7, SEQ ID NO:9, SEQ ID NO: 11, or SEQ ID NO: 13, and the polynucleotide encoding the light chain polypeptide of the anti-CD47/anti-CTLA-4 bispecific antigen-binding protein or the fragment thereof includes a sequence that is at least 80%, 85%, 90%, 95%, 96%, 97%, 98%, or 99% identical to the nucleotide sequence as set forth in SEQ ID NO:5. In some specific embodiments, the polynucleotide encoding the heavy chain fusion protein of the anti-CD47/anti-CTLA-4 bispecific antigen-binding protein or the fragment thereof includes the nucleotide sequence as set forth in SEQ ID NO:7, SEQ ID NO:9, SEQ ID NO: 11, or SEQ ID NO:13, and the polynucleotide encoding the light chain polypeptide of the anti-CD47/anti-CTLA-4 bispecific antigen-binding protein or the fragment thereof includes the nucleotide sequence as set forth in SEQ ID NO:5. In some embodiments, the polynucleotide encoding the light chain fusion protein of the anti-CD47/anti-CTLA-4 bispecific antigen-binding protein or the fragment thereof includes a sequence that is at least 80%, 85%, 90%, 95%, 96%, 97%, 98%, or 99% identical to the nucleotide sequence as set forth in SEQ ID NO:15 or SEQ ID NO:17, and the polynucleotide encoding the heavy chain of the anti-CD47/anti-CTLA-4 bispecific antigen-binding protein or the fragment thereof includes a sequence that is at least 80%, 85%, 90%, 95%, 96%, 97%, 98%, or 99% identical to the nucleotide sequence as set forth in SEQ ID NO:3. In some specific embodiments, the polynucleotide encoding the light chain fusion protein of the anti-CD47/anti-CTLA-4 bispecific antigen-binding protein or the fragment thereof includes the nucleotide sequence as set forth in SEQ ID NO: 15 or SEQ ID NO: 17, and the polynucleotide encoding the heavy chain polypeptide of the anti-CD47/anti-CTLA-4 bispecific antigen-binding protein or the fragment thereof includes the nucleotide sequence as set forth in SEQ ID NO:3.

Further, a vector including the isolated polynucleotide encoding the isolated anti-CD47/anti-CTLA-4 bispecific antigen-binding protein or the fragment thereof is provided. It can be commonly known to those skilled in the art that a vector is a plasmid, a phage vector, or a viral vector. In some specific embodiments, the vector is a recombinant expression vector, for example, a plasmid. These vectors include various elements to support the functions thereof as conventional expression vectors, for example, including promoters, ribosome binding elements, terminators, enhancers, selective markers, and origins of replication. The promoters may be conventional promoters, inducible promoters, or repressible promoters. It can be commonly known in the art that many expression vectors can deliver nucleic acids into cells and can be used to produce antibodies or antigen-binding fragments thereof in cells. According to the method in the examples of the present invention, conventional cloning techniques or artificial gene synthesis can be used to produce recombinant expression vectors.

Further, a host cell including the isolated polynucleotide or the vector is provided. In the present invention, any host cell conventional in the art can be used for the expression of antibodies or antigen-binding fragments thereof. In some embodiments, the host cell is *E. coli* TG1 or BL21 (used to express scFv or Fab antibodies), CHO-DG44, CHO-3E7, CHO-K1, or HEK293. According to specific examples, the recombinant expression vector is transfected into the host cell by a conventional method (such as chemical transfection, thermal transfection, or electro-transfection), and is stably integrated into the host cell genome, so that the recombinant nucleic acids can be effectively expressed.

According to another aspect, the present invention provides a method for producing an isolated anti-CD47/anti-CTLA-4 bispecific antigen-binding protein or a fragment thereof, including culturing the host cell including the polynucleotide encoding the bispecific antigen-binding protein or the fragment thereof in the present invention under proper conditions, and recovering an antibody or a fragment thereof from the cell or a cell culture medium. The expression antibody or the fragment thereof may be obtained from cells or extracted and purified by using conventional methods in the art.

According to another aspect, the present invention provides a pharmaceutical composition, including the isolated anti-CD47/anti-CTLA-4 bispecific antigen-binding protein or the fragment thereof and a pharmaceutically acceptable carrier. The "pharmaceutically acceptable carrier" refers to a solid or liquid diluent, a filler, an antioxidant, a stabilizer, or other substances that can be administered safely. These substances are suitable for human and/or animal administration without excessive side effects, and are suitable for maintaining the activity of drugs or active agents therein. Different carriers well known in the art may be administered according to the route of administration, including, but not limited to, carbohydrates, starch, cellulose and derivatives thereof, maltose, gelatin, talc, calcium sulfate, vegetable oils, synthetic oils, polyols, alginic acid, phosphate buffers, emulsifiers, isotonic saline, and/or pyrogen-free water. The pharmaceutical composition provided in the present invention may be prepared into clinically acceptable dosage forms such as powders and injections. Any proper route may be used to administer the pharmaceutical composition of the present invention to subjects, for example, it may be administered by oral, intravenous infusion, intramuscular injection, subcutaneous injection, subperitoneal, rectal, sublingual, inhalation, or transdermal.

According to another aspect, the present invention provides a method for treating subjects suffering from or at risk of suffering from diseases related to abnormal expression of CD47 and/or CTLA-4, including administering the pharmaceutical composition with an effective amount to the subjects.

According to another aspect, the present invention provides use of the anti-CD47/anti-CTLA-4 bispecific antigen-binding protein or the fragment thereof, the polynucleotide, the vector, and the host cell in preparing medicines for diseases related to abnormal expression of CD47 and/or CTLA-4.

In some embodiments, the cancers are solid tumors, such as colorectal cancer, non-small cell lung cancer, small cell lung cancer, renal cell cancer, ovarian cancer, breast cancer, pancreatic cancer, stomach cancer, bladder cancer, esophageal cancer, mesothelioma, melanoma, head and neck cancer, Hodgkin lymphoma, hepatocellular carcinoma, advanced kidney cancer, thyroid cancer, endometrial cancer, prostate cancer, urothelial carcinoma, and cervical cancer. In a preferred embodiment, the cancers are solid tumors, such as pancreatic cancer, non-small cell lung cancer, melanoma, breast cancer, stomach cancer, colorectal cancer, head and neck cancer, Hodgkin lymphoma, hepatocellular carcinoma, advanced kidney cancer, thyroid cancer, ovarian cancer, endometrial cancer, prostate cancer, urothelial carcinoma, and cervical cancer.

In some embodiments, the method further includes administering additional tumor treatment to the subjects, such as surgery, radiation therapy, chemotherapy, immunotherapy, hormone therapy, or a combination thereof.

In the present invention, the CTLA-4 sdAb is linked to the end of the heavy chain/light chain of the anti-CD47 monoclonal antibody in a specific way, and the produced anti-CD47/anti-CTLA-4 bispecific antigen-binding protein can block two ways of tumor immune escape. Compared with the anti-CD47 antibody, the bispecific antigen-binding protein with the sdAb linked to the N-terminus of the antibody has reduced affinity to the CD47 antigen, which may reduce the clinical red blood cell toxicity caused by binding to normal red blood cells to a certain extent.

### Explanation of Terms

An "antigen-binding protein fragment" means a fragment of an antibody and an antibody mimetic, generally including at least part of the antigen binding regions or variable regions (for example, one or more CDRs) of a parental antibody. The antibody fragment retains at least some of the binding specificity of the parental antibody. For example, the antigen-binding protein fragment that can bind to CD47 or part of it includes, but is not limited to, sdAb, Fab (for example, obtained by papain digestion of antibodies), F(ab')₂ (for example, obtained by pepsin digestion), and Fv or scFv (for example, obtained by molecular biology techniques).

"sdAb" refers to single antigen-binding polypeptide with three CDRs. The sdAb can bind to the antigen independently without pairing with corresponding CDR-containing polypeptide. In some cases, sdAb is artificially engineered from camelid heavy chain antibodies and is referred to as a "V_{H}H domain". Cartilaginous fishes also have heavy chain antibodies (immunoglobulin new antigen receptor (IgNAR)), and sdAb referred to as a "V_{NAR} domain" may also be produced from this class of antibodies. Camelid sdAb is a smallest known antigen-binding antibody fragment (Refer to *e.g.,* Hamers-Casterman et al., Nature 363:446-8 (1993); Greenberg et al., Nature 374:168-73 (1995); Hassanzadeh-Ghassabeh et al., Nanomedicine (Lond), 8:1013-26 (2013)). The basic V_{H}H has the following structure from the N-terminus to the C-terminus: FR1-CDR1-FR2-CDR2-FR3-CDR3-FR4, where FR1 to FR4 are framework regions 1 to 4, and CDR1 to CDR3 are CDRs 1 to 3. The anti-CTLA-4 sdAb in the present invention refers to sdAb that can specifically bind to CTLA-4, in particular, sdAb that can bind to human CTLA-4. The anti-CTLA-4 sdAb in the present invention may be selected from the anti-CTLA-4 sdAb specifically described in the patent application WO2018068695A1. The construction, expression, extraction, and purification methods of the anti-CTLA-4 sdAb in the present invention may refer to the patent application WO2018068695A1.

A "full-length antibody" refers to an antibody having four full-length chains, including heavy chains and light chains containing Fc regions. The anti-CD47 antibody in the present invention refers to an antibody that can specifically bind to CD47, in particular, an antibody that can bind to human CD47. The anti-CD47 antibody in the present invention may be selected from the anti-CD47 antibody specifically described in PCT/CN2019/072929. The construction, expression, extraction, and purification methods of the anti-CD47 antibody in the present invention may refer to the patent application PCT/CN2019/072929.

A "mutation" is an alteration of one or more (several) amino acid residues at one or more (several) locations included in an antigen-binding protein or a protein fragment, that is, the substitution, insertion, and/or deletion of polypeptide. The substitution refers to substituting different amino acids for the amino acid occupying a certain location. The deletion refers to deleting the amino acid occupying a certain location. The insertion refers to refers to inserting 1 to 5 amino acids after the amino acid occupying a certain location.

The "amino acid sequence identity" is defined as the percentage of amino acid residues in a candidate sequence identical to the amino acid residues in a specific peptide or polypeptide sequence after the sequences are compared and gaps are introduced when necessary to obtain the maximum percent sequence identity without considering any conservative substitutions as part of the sequence identity. Sequence comparison can be performed in a variety of ways within the skill of the art to determine percent amino acid sequence identity, for example, publicly available computer software such as BLAST, BLAST-2, ALIGN or Megalign (DNASTAR) software is used. Those skilled in the art can determine appropriate parameters for measuring the comparison, including any algorithm required to obtain the maximum comparison over the full length of the compared sequences.

A "GS linker" refers to the GS combination of glycine (G) and serine (S), and is used to link a plurality of proteins together to form a fusion protein. The commonly used GS combination is (GGGGS)n, which changes the length of the linker sequence by changing n, and most of the GS combination is (GGGGS)3. In addition, glycine and serine may also produce different linker sequences through other combinations, for example, the GS combination of G15-linker used in the present invention is GGGGSGGGSGGGGS, and the GS combination of G9-linker is GGGGSGGGS.

### BRIEF DESCRIPTION OF THE DRAWINGS

FIG. 1 shows the affinity between a sample and CHO-K1 cells expressing human CTLA-4 measured by a flow cytometer.
FIG. 2 shows the affinity between a sample and CHO-K1 cells expressing CD47 measured by a flow cytometer.
FIG. 3 shows the CTLA-4 blocking activity of a sample measured by a CTLA-4 blocking bioassay system.
FIG. 4 shows the activity of the bispecific antibody CTLA4-G15-HC tested by a cell phagocytosis experiment of the anti-CD47 antibody.

### DETAILED DESCRIPTION

The present invention is described in detail below with reference to specific implementations. It should be understood that these implementations are merely intended to describe the present invention rather than to limit the scope of the present invention. In addition, it should be understood that, after reading the teaching of the present invention, those skilled in the art may make various changes or modifications to the present invention, and these equivalent forms also fall within the scope defined by the appended claims of this application. Unless otherwise specified, the methods and materials in the examples described below are commercially available and conventional products.

### Example 1 Construction and expression of anti-CD47/anti-CTLA-4 bispecific antibody

A series of anti-CD47/anti-CTLA-4 bispecific antibodies were designed by using an anti-CD47 monoclonal antibody (mAb) (the CDR, full-length nucleotide, and amino acid sequence of the antibody are shown in Table 1) and a CTLA-4 sdAb (the CDR, full-length nucleotide, and amino acid sequence of the antibody are shown in Table 2). The CTLA-4 sdAb was fused to the N-terminus or C-terminus of the heavy chain or light chain of the anti-CD47 mAb by using three linker sequences (E-linker: EPKSSDKTHTSPPSP, G15-linker: GGGGSGGGGSGGGGS, or G9-linker: GGGGSGGGS). Each bispecific antibody structure was composed of two identical fused polypeptide chains and two identical natural polypeptide chains, the DNA sequence expressing each polypeptide chain was inserted into the pTT5 vector between EcoRI and HindIII restriction sites. Each plasmid also includes a secretion signal sequence of a protein secreted into a growth medium. The CTLA-4 sdAb was fused to the N-terminus of IgG4-Fc with site mutation (S228P and L235E), as a control for biological activity measurement *in vitro.* The plasmids expressing the bispecific antibody protein are shown in Table 3.

**Table 1. DNA and amino acid sequences of anti-CD47 mAb**

| | **DNA sequence** | **SEQ NO: ID** | | | | |
|---|---|---|---|---|---|---|
| **Heavy chain DNA sequence of anti-CD47 antibody** | GAGGTGCAGCTGGTGCAGTCCGGAGCTGAGGTGAAGA AGCCAGGATCCAGCGTGAAGGTGAGCTGCAAGGCTAG CGGCTACTCTTTCACCCACCATTGGATCCACTGGGTGAG GCAGGCTCCTGGACAGGGACTGGAGTGGATGGGCATGA TCGACGCTTCCGATAGCGAGACAAGACTGTCTCAGAAG TTTAAGGACCGCGTGACCATCACAGCCGATAAGTCTACC TCCACAGCTTACATGGAGCTGTCTTCCCTGAGATCCGAG GACACCGCCGTGTACTATTGTGCTAGGCTGGGCCGGTAC TATTTCGATTATTGGGGCCAGGGCACCACAGTGACAGTG AGCTCTGCCAGCACAAAGGGCCCTTCCGTGTTCCCACT GGCTCCCTGCTCCAGAAGCACATCTGAGTCCACCGCCG CTCTGGGCTGTCTGGTGAAGGACTACTTCCCTGAGCCA GTGACCGTGTCCTGGAACAGCGGCGCCCTGACATCTGG CGTGCACACCTTTCCAGCTGTGCTGCAGTCCAGCGGCC | 3 | | | | |
| | TGTACTCCCTGTCTTCCGTGGTGACAGTGCCCAGCTCTT CCCTGGGCACCAAGACATATACCTGCAACGTGGACCAT AAGCCTTCCAATACCAAGGTGGATAAGAGGGTGGAGAG CAAGTACGGACCACCTTGCCCACCATGTCCAGCTCCTG AGTTTGAGGGAGGACCATCCGTGTTCCTGTTTCCTCCAA AGCCTAAGGACACCCTGATGATCAGCCGGACACCTGAG GTGACCTGCGTGGTGGTGGACGTGTCTCAGGAGGATCC AGAGGTGCAGTTCAACTGGTACGTGGATGGCGTGGAGG TGCACAATGCTAAGACCAAGCCAAGAGAGGAGCAGTTT AATTCCACATACCGCGTGGTGAGCGTGCTGACCGTGCT GCATCAGGATTGGCTGAACGGCAAGGAGTATAAGTGCA AGGTGTCCAATAAGGGCCTGCCCAGCTCTATCGAGAAG ACAATCAGCAAGGCTAAGGGACAGCCTAGGGAGCCAC AGGTGTACACCCTGCCCCCTTCTCAGGAGGAGATGACA AAGAACCAGGTGTCCCTGACCTGTCTGGTGAAGGGCTT CTATCCAAGCGACATCGCTGTGGAGTGGGAGTCTAATG GCCAGCCCGAGAACAATTACAAGACCACACCACCCGTG CTGGACTCTGATGGCTCCTTCTTTCTGTATTCTAGGCTGA CAGTGGATAAGTCCCGGTGGCAGGAGGGCAACGTGTTT AGCTGCTCTGTGATGCACGAGGCCCTGCACAATCATTAT ACCCAGAAGTCCCTGAGCCTGTCTCTGGGCAAG | | | | | |
| **Heavy chain amino acid sequence H0 of anti-CD47 antibody** | EVQLVQSGAEVKKPGSSVKVSCKASGYSFTHHWIHWVR QAPGQGLEWMGMIDASDSETRLSQKFKDRVTITADKSTS TAYMELSSLRSEDTAVYYCARLGRYYFDYWGQGTTVTVS SASTKGPSVFPLAPCSRSTSESTAALGCLVKDYFPEPVTVS WNSGALTSGVHTFPAVLQSSGLYSLSSVVTVPSSSLGTKTY TCNVDHKPSNTKVDKRVESKYGPPCPPCPAPEFEGGPSVF LFPPKPKDTLMISRTPEVTCVVVDVSQEDPEVQFNWYVD GVEVHNAKTKPREEQFNSTYRVVSVLTVLHQDWLNGKE YKCKVSNKGLPSSIEKTISKAKGQPREPQVYTLPPSQEEM TKNQVSLTCLVKGFYPSDIAVEWESNGQPENNYKTTPPVL DSDGSFFLYSRLTVDKSRWQEGNVFSCSVMHEALHNHYT QKSLSLSLGK | 4 | | | | |
| **Light chain DNA sequence of anti-CD47 antibody** | GAGATCGTGCTGACCCAGTCTCCAGCCACACTGTCTCT GTCCCCAGGAGAGAGGGCCACCCTGAGCTGCCGGGCTT CTGAGAACGTGGGCACATACATCTCCTGGTATCAGCAG AAGCCAGGACAGGCTCCTAGGCTGCTGATCTACGGCGC TAGCAATAGATATACCGGCATCCCTGCTCGCTTCAGCGG ATCTGGATCCGGCACAGACTTTACCCTGACAATCTCCAG CCTGGAGCCAGAGGATTTCGCCGTGTACTATTGTGGCGA GTCCTACGGCCACCTGTATACCTTTGGCGGCGGCACAA AGGTGGAGATCAAGCGAACGGTGGCTGCACCATCTGTC TTCATCTTCCCGCCATCTGATGAGCAGTTGAAATCTGGA ACTGCCTCTGTTGTGTGCCTGCTGAATAACTTCTATCCC AGAGAGGCCAAAGTACAGTGGAAGGTGGATAACGCCC TCCAATCGGGTAACTCCCAGGAGAGTGTCACAGAGCAG GACAGCAAGGACAGCACCTACAGCCTCAGCAGCACCC TGACGCTGAGCAAAGCAGACTACGAGAAACACAAAGT CTACGCCTGCGAAGTCACCCATCAGGGCCTGAGCTCGC | 5 | | | | |
| | CCGTCACAAAGAGCTTCAACAGGGGAGAGTGT | | | | | |
| **Light chain amino acid sequence L0** of **anti-CD47 antibody** | EIVLTQSPATLSLSPGERATLSCRASENVGTYISWYQQKPG QAPRLLIYGASNRYTGIPARFSGSGSGTDFTLTISSLEPEDF AVYYCGESYGHLYTFGGGTKVEIKRTVAAPSVFIFPPSDEQ LKSGTASVVCLLNNFYPREAKVQWKVDNALQSGNSQES VTEQDSKDSTYSLSSTLTLSKADYEKHKVYACEVTHQGLS SPVTKSFNRGEC | | | | | 6 |

| | **CDR1 sequence** | **SEQ ID NO:** | **CDR2 sequence** | **SEQ ID NO:** | **CDR3 sequence** | **SEQ ID NO:** |
|---|---|---|---|---|---|---|
| **Heavy chain CDR sequence of anti-CD47 antibody** | GYSFTHH WIH | 21 | MIDASDSET RLSQKFKD | 22 | LGRYYFD Y | 23 |
| **Light chain CDR sequence of anti-CD47 antibody** | RASENVG TYIS | 24 | GASNRYT | 25 | GESYGHL YT | 26 |

**Table 2. DNA and amino acid sequences of CTLA-4 sdAb**

| | **Sequence** | | | | | **SEQ ID NO:** |
|---|---|---|---|---|---|---|
| **DNA sequence of CTLA-4 sdAb** | GAGGTGCAGCTGGTGGAATCTGGGGGAGGACTGGTGC AGCCTGGGGGAAGCC TGAGACTGAGTTGTGCCGCAAGCGGATACACTTACAGC AGGCACTGCCTGGGA TGGTTCCGACAGGCACCAGGCAAGGGACGAGAGGCCG TGTCTACCATCGACAG TGATGGGAGCACATCCTACGCTGACTCAGTCAAGGGAC GGTTTACTATCAGCAG AGATAACGCAAAGAACACCCTGTATCTGCAGATGAACT CCCTGAGACCAGAAGA CACAGCCGTGTACTATTGCGCTATCGGACCCAACCCTCG GTACTGTTCTGGCGCC CCCAATACCCGGGGAGCCGAACATTACTTTGGGTATTGG GGACAGGGCACACTG GTGACCGTGAGCAGC | | | | | 27 |
| **Amino acid sequence of CTLA-4 sdAb** | EVQLVESGGGLVQPGGSLRLSCAASGYTYSRHCLGWFRQ APGKGREAVSTIDSDGS TSYADSVKGRFTISRDNAKNTLYLQMNSLRPEDTAVYYC AIGPNPRYCSGAPNTRG AEHYFGYWGQGTLVTVSS | | | | | 28 |

| | **CDR1 sequence** | **SEQ ID NO:** | **CDR2 sequence** | **SEQ ID NO:** | **CDR3 sequence** | **SEQ ID NO:** |
|---|---|---|---|---|---|---|
| **CDR amino acid sequence of CTLA-4 sdAb** | GYTYSRH CLG | 29 | TIDSDGSTS YADSVKG | 30 | GPNPRYCS GAPN TRGAEHYF GY | 31 |

After the expression plasmids were transfected into CHO-3E7 host cells, the resulting host cells were cultured in an incubator at 37°C and 100 rpm for 6 days. A supernatant was extracted by centrifugation, and a bispecific antibody protein was purified with a Protein A column.

As described above, the CD47 mAb is composed of the heavy chain H0 and the light chain L0. The CTLA-4 sdAb was fused to the N-terminus or C-terminus of the heavy chain or light chain of the CD47 mAb by three linker sequences (E-linker: EPKSSDKTHTSPPSP, G15-linker: GGGGSGGGGSGGGGS, or G9-linker: GGGGSGGGS) to produce a series of different bispecific antibodies. First, the E-linker sequence was used to construct the following fusion proteins: (1). the CTLA-4 sdAb was fused to the C-terminus of the heavy chain H0 to produce a new polypeptide referred to as H1; and (2). the CTLA-4 sdAb was fused to the N-terminus of the heavy chain H0 to produce a new polypeptide referred to as H2. Similarly, the G15-linker sequence was used to construct the following fusion proteins: (1). the CTLA-4 sdAb was fused to the C-terminus of the heavy chain H0 to produce a new polypeptide referred to as H3; (2). the CTLA-4 sdAb was fused to the N-terminus of the heavy chain H0 to produce a new polypeptide referred to as H4; and (3). the CTLA-4 sdAb was fused to the N-terminus of the light chain L0 to produce a new polypeptide referred to as L1. Then, by using the G9-linker sequence, the CTLA-4 sdAb was fused to the N-terminus of the light chain L0 to produce a new polypeptide referred to as L2.

These constructed heavy chain fusion proteins H1, H2, H3, and H4 were separately combined with the unmodified parental light chain polypeptide chain L0, or these constructed light chain fusion proteins L1 and L2 were separately combined with the unmodified heavy chain polypeptide chain H0, to produce a series of bispecific antibodies. The heavy chain fusion protein H1 was combined with the parental light chain L0 to produce a bispecific antibody CTLA4-E-HC. The heavy chain fusion protein H2 was combined with the parental light chain L0 to produce a bispecific antibody CTLA4-E-HN. The heavy chain fusion protein H3 was combined with the parental light chain L0 to produce a bispecific antibody CTLA4-G15-HC. The heavy chain fusion protein H4 was combined with the parental light chain L0 to produce a bispecific antibody CTLA4-G15-HN. The light chain fusion protein L1 was combined with the parental heavy chain H0 to produce a bispecific antibody CTLA4-G15-LN. The light chain fusion protein L2 was combined with the parental heavy chain H0 to produce a bispecific antibody CTLA4-G9-LN.

The human IGg4-Fc was modified by site mutation (S228P and L235E), and then the CTLA-4 sdAb was linked to the N-terminus of the modified IGg4-Fc, to produce a new fusion protein H5, to further construct the Fc fusion protein sdAb-CTLA4-IgG4PE.

**Table 3. Plasmids and proteins for construction of bispecific antibody**

| Protein | Component | Plasmid | Amino acid SEQ ID NO: |
|---|---|---|---|
| CD47 | H0 | pTT5-CD47HC | 4 |
| | L0 | pTT5-CD47LC | 6 |
| CTLA4-E-HC | H1 | pTT5-CD47HC-E-CTLA4 | 8 |
| | L0 | pTT5-CD47LC | 6 |
| CTLA4-E-HN | H2 | pTT5-CTLA4-E-CD47HC | 10 |
| | L0 | pTT5-CD47LC | 6 |
| CTLA4-G15-HC | H3 | pTT5-CD47HC-G15-CTLA4 | 12 |
| | L0 | pTT5-CD47LC | 6 |
| CTLA4-G15-HN | H4 | pTT5-CTLA4-G15-CD47HC | 14 |
| | L0 | pTT5-CD47LC | 6 |
| CTLA4-G15-LN | L1 | pTT5-CTLA4-G15-CD47LC | 16 |
| | H0 | pTT5-CD47HC | 4 |
| CTLA4-G9-LN | L2 | pTT5-CTLA4-G9-CD47LC | 18 |
| | H0 | pTT5-CD47HC | 4 |
| sdab-CTLA4-IgG4PE | H5 | pTT5-sdab-CTLA4-IgG4PE | 20 |

DNA sequence of secretion signal peptide (SEQ ID NO: 1) 
ATGGGCTGGTCCTGCATCATCCTGTTCCTGGTGGCTACCGCCACCGGCGTGCA
CTCC
Amino acid sequence of secretion signal peptide (SEQ ID NO: 2)
   MGWSCIILFLVATATGVHS
DNA sequence of polypeptide chain H0 (SEQ ID NO: 3) 
GAGGTGCAGCTGGTGCAGTCCGGAGCTGAGGTGAAGAAGCCAGGATCCAGC
GTGAAGGTGAGCTGCAAGGCTAGCGGCTACTCTTTCACCCACCATTGGATCCACT
GGGTGAGGCAGGCTCCTGGACAGGGACTGGAGTGGATGGGCATGATCGACGCTT
CCGATAGCGAGACAAGACTGTCTCAGAAGTTTAAGGACCGCGTGACCATCACAGC
CGATAAGTCTACCTCCACAGCTTACATGGAGCTGTCTTCCCTGAGATCCGAGGACA
 
CCGCCGTGTACTATTGTGCTAGGCTGGGCCGGTACTATTTCGATTATTGGGGCCAG
GGCACCACAGTGACAGTGAGCTCTGCCAGCACAAAGGGCCCTTCCGTGTTCCCA
CTGGCTCCCTGCTCCAGAAGCACATCTGAGTCCACCGCCGCTCTGGGCTGTCTGG
TGAAGGACTACTTCCCTGAGCCAGTGACCGTGTCCTGGAACAGCGGCGCCCTGAC
ATCTGGCGTGCACACCTTTCCAGCTGTGCTGCAGTCCAGCGGCCTGTACTCCCTGT
CTTCCGTGGTGACAGTGCCCAGCTCTTCCCTGGGCACCAAGACATATACCTGCAA
CGTGGACCATAAGCCTTCCAATACCAAGGTGGATAAGAGGGTGGAGAGCAAGTAC
GGACCACCTTGCCCACCATGTCCAGCTCCTGAGTTTGAGGGAGGACCATCCGTGT
TCCTGTTTCCTCCAAAGCCTAAGGACACCCTGATGATCAGCCGGACACCTGAGGT
GACCTGCGTGGTGGTGGACGTGTCTCAGGAGGATCCAGAGGTGCAGTTCAACTG
GTACGTGGATGGCGTGGAGGTGCACAATGCTAAGACCAAGCCAAGAGAGGAGCA
GTTTAATTCCACATACCGCGTGGTGAGCGTGCTGACCGTGCTGCATCAGGATTGGC
TGAACGGCAAGGAGTATAAGTGCAAGGTGTCCAATAAGGGCCTGCCCAGCTCTAT
CGAGAAGACAATCAGCAAGGCTAAGGGACAGCCTAGGGAGCCACAGGTGTACAC
CCTGCCCCCTTCTCAGGAGGAGATGACAAAGAACCAGGTGTCCCTGACCTGTCTG
GTGAAGGGCTTCTATCCAAGCGACATCGCTGTGGAGTGGGAGTCTAATGGCCAGC
CCGAGAACAATTACAAGACCACACCACCCGTGCTGGACTCTGATGGCTCCTTCTT
TCTGTATTCTAGGCTGACAGTGGATAAGTCCCGGTGGCAGGAGGGCAACGTGTTT
AGCTGCTCTGTGATGCACGAGGCCCTGCACAATCATTATACCCAGAAGTCCCTGAG
CCTGTCTCTGGGCAAG
Amino acid sequence of polypeptide chain H0 (SEQ ID NO: 4) 
EVQLVQSGAEVKKPGSSVKVSCKASGYSFTHHWIHWVRQAPGQGLEWMGMID
ASDSETRLSQKFKDRVTITADKSTSTAYMELSSLRSEDTAVYYCARLGRYYFDYWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCPP
CPAPEFEGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGK
DNA sequence of polypeptide chain L0 (SEQ ID NO: 5) 
GAGATCGTGCTGACCCAGTCTCCAGCCACACTGTCTCTGTCCCCAGGAGAGA
GGGCCACCCTGAGCTGCCGGGCTTCTGAGAACGTGGGCACATACATCTCCTGGTA
TCAGCAGAAGCCAGGACAGGCTCCTAGGCTGCTGATCTACGGCGCTAGCAATAGA
TATACCGGCATCCCTGCTCGCTTCAGCGGATCTGGATCCGGCACAGACTTTACCCT
 
GACAATCTCCAGCCTGGAGCCAGAGGATTTCGCCGTGTACTATTGTGGCGAGTCCT
ACGGCCACCTGTATACCTTTGGCGGCGGCACAAAGGTGGAGATCAAGCGAACGGT
GGCTGCACCATCTGTCTTCATCTTCCCGCCATCTGATGAGCAGTTGAAATCTGGAA
CTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGC
AGGACAGCAAGGACAGCACCTACAGCCTCAGCAGCACCCTGACGCTGAGCAAAG
CAGACTACGAGAAACACAAAGTCTACGCCTGCGAAGTCACCCATCAGGGCCTGA
GCTCGCCCGTCACAAAGAGCTTCAACAGGGGAGAGTGT
Amino acid sequence of polypeptide chain L0 (SEQ ID NO: 6) 
EIVLTQSPATLSLSPGERATLSCRASENVGTYISWYQQKPGQAPRLLIYGASNRYT
GIPARFSGSGSGTDFTLTISSLEPEDFAVYYCGESYGHLYTFGGGTKVEIKRTVAAPSVF
IFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC
DNA sequence of polypeptide chain H1 (SEQ ID NO: 7) 
GAGGTGCAGCTGGTGCAGTCCGGAGCTGAGGTGAAGAAGCCAGGATCCAGC
GTGAAGGTGAGCTGCAAGGCTAGCGGCTACTCTTTCACCCACCATTGGATCCACT
GGGTGAGGCAGGCTCCTGGACAGGGACTGGAGTGGATGGGCATGATCGACGCTT
CCGATAGCGAGACAAGACTGTCTCAGAAGTTTAAGGACCGCGTGACCATCACAGC
CGATAAGTCTACCTCCACAGCTTACATGGAGCTGTCTTCCCTGAGATCCGAGGACA
CCGCCGTGTACTATTGTGCTAGGCTGGGCCGGTACTATTTCGATTATTGGGGCCAG
GGCACCACAGTGACAGTGAGCTCTGCCAGCACAAAGGGCCCTTCCGTGTTCCCA
CTGGCTCCCTGCTCCAGAAGCACATCTGAGTCCACCGCCGCTCTGGGCTGTCTGG
TGAAGGACTACTTCCCTGAGCCAGTGACCGTGTCCTGGAACAGCGGCGCCCTGAC
ATCTGGCGTGCACACCTTTCCAGCTGTGCTGCAGTCCAGCGGCCTGTACTCCCTGT
CTTCCGTGGTGACAGTGCCCAGCTCTTCCCTGGGCACCAAGACATATACCTGCAA
CGTGGACCATAAGCCTTCCAATACCAAGGTGGATAAGAGGGTGGAGAGCAAGTAC
GGACCACCTTGCCCACCATGTCCAGCTCCTGAGTTTGAGGGAGGACCATCCGTGT
TCCTGTTTCCTCCAAAGCCTAAGGACACCCTGATGATCAGCCGGACACCTGAGGT
GACCTGCGTGGTGGTGGACGTGTCTCAGGAGGATCCAGAGGTGCAGTTCAACTG
GTACGTGGATGGCGTGGAGGTGCACAATGCTAAGACCAAGCCAAGAGAGGAGCA
GTTTAATTCCACATACCGCGTGGTGAGCGTGCTGACCGTGCTGCATCAGGATTGGC
TGAACGGCAAGGAGTATAAGTGCAAGGTGTCCAATAAGGGCCTGCCCAGCTCTAT
CGAGAAGACAATCAGCAAGGCTAAGGGACAGCCTAGGGAGCCACAGGTGTACAC
CCTGCCCCCTTCTCAGGAGGAGATGACAAAGAACCAGGTGTCCCTGACCTGTCTG
 
GTGAAGGGCTTCTATCCAAGCGACATCGCTGTGGAGTGGAGTCTAATGGCCAGC
CCGAGAACAATTACAAGACCACACCACCCGTGCTGGACTCTGATGGCTCCTTCTT
TCTGTATTCTAGGCTGACAGTGGATAAGTCCCGGTGGCAGGAGGGCAACGTGTTT
AGCTGCTCTGTGATGCACGAGGCCCTGCACAATCATTATACCCAGAAGTCCCTGAG
CCTGTCTCTGGGCAAGGAACCTAAGTCTAGCGACAAAACTCATACCAGCCCCCCT
AGTCCAGAGGTGCAGCTGGTGGAATCTGGGGGAGGACTGGTGCAGCCTGGGGGA
AGCCTGAGACTGAGTTGTGCCGCAAGCGGATACACTTACAGCAGGCACTGCCTGG
GATGGTTCCGACAGGCACCAGGCAAGGGACGAGAGGCCGTGTCTACCATCGACA
GTGATGGGAGCACATCCTACGCTGACTCAGTCAAGGGACGGTTTACTATCAGCAG
AGATAACGCAAAGAACACCCTGTATCTGCAGATGAACTCCCTGAGACCAGAAGAC
ACAGCCGTGTACTATTGCGCTATCGGACCCAACCCTCGGTACTGTTCTGGCGCCCC
CAATACCCGGGGAGCCGAACATTACTTTGGGTATTGGGGACAGGGCACACTGGTG
ACCGTGAGCAGC
Amino acid sequence of polypeptide chain H1 (SEQ ID NO: 8) 
EVQLVQSGAEVKKPGSSVKVSCKASGYSFTHHWIHWVRQAPGQGLEWMGMID
ASDSETRLSQKFKDRVTITADKSTSTAYMELSSLRSEDTAVYYCARLGRYYFDYWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCPP
CPAPEFEGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGKEPKSSDKTHT
SPPSPEVQLVESGGGLVQPGGSLRLSCAASGYTYSRHCLGWFRQAPGKGREAVSTIDS
DGSTSYADSVKGRFTISRDNAKNTLYLQMNSLRPEDTAVYYCAIGPNPRYCSGAPNTR
GAEHYFGYWGQGTLVTVSS
DNA sequence of polypeptide chain H2 (SEQ ID NO: 9) 
GAGGTGCAGCTGGTGGAATCTGGGGGAGGACTGGTGCAGCCTGGGGGAAGC
CTGAGACTGAGTTGTGCCGCAAGCGGATACACTTACAGCAGGCACTGCCTGGGAT
GGTTCCGACAGGCACCAGGCAAGGGACGAGAGGCCGTGTCTACCATCGACAGTG
ATGGGAGCACATCCTACGCTGACTCAGTCAAGGGACGGTTTACTATCAGCAGAGA
TAACGCAAAGAACACCCTGTATCTGCAGATGAACTCCCTGAGACCAGAAGACACA
GCCGTGTACTATTGCGCTATCGGACCCAACCCTCGGTACTGTTCTGGCGCCCCCAA
TACCCGGGGAGCCGAACATTACTTTGGGTATTGGGGACAGGGCACACTGGTGACC
GTGAGCAGCGAACCTAAGTCTAGCGACAAAACTCATACCAGCCCCCCTAGTCCAG
 
AGGTGCAGCTGGTGCAGTCCGGAGCTGAGGTGAAGAAGCCAGGATCCAGCGTGA
AGGTGAGCTGCAAGGCTAGCGGCTACTCTTTCACCCACCATTGGATCCACTGGGT
GAGGCAGGCTCCTGGACAGGGACTGGAGTGGATGGGCATGATCGACGCTTCCGAT
AGCGAGACAAGACTGTCTCAGAAGTTTAAGGACCGCGTGACCATCACAGCCGATA
AGTCTACCTCCACAGCTTACATGGAGCTGTCTTCCCTGAGATCCGAGGACACCGC
CGTGTACTATTGTGCTAGGCTGGGCCGGTACTATTTCGATTATTGGGGCCAGGGCA
CCACAGTGACAGTGAGCTCTGCCAGCACAAAGGGCCCTTCCGTGTTCCCACTGGC
TCCCTGCTCCAGAAGCACATCTGAGTCCACCGCCGCTCTGGGCTGTCTGGTGAAG
GACTACTTCCCTGAGCCAGTGACCGTGTCCTGGAACAGCGGCGCCCTGACATCTG
GCGTGCACACCTTTCCAGCTGTGCTGCAGTCCAGCGGCCTGTACTCCCTGTCTTCC
GTGGTGACAGTGCCCAGCTCTTCCCTGGGCACCAAGACATATACCTGCAACGTGG
ACCATAAGCCTTCCAATACCAAGGTGGATAAGAGGGTGGAGAGCAAGTACGGACC
ACCTTGCCCACCATGTCCAGCTCCTGAGTTTGAGGGAGGACCATCCGTGTTCCTGT
TTCCTCCAAAGCCTAAGGACACCCTGATGATCAGCCGGACACCTGAGGTGACCTG
CGTGGTGGTGGACGTGTCTCAGGAGGATCCAGAGGTGCAGTTCAACTGGTACGTG
GATGGCGTGGAGGTGCACAATGCTAAGACCAAGCCAAGAGAGGAGCAGTTTAAT
TCCACATACCGCGTGGTGAGCGTGCTGACCGTGCTGCATCAGGATTGGCTGAACG
GCAAGGAGTATAAGTGCAAGGTGTCCAATAAGGGCCTGCCCAGCTCTATCGAGAA
GACAATCAGCAAGGCTAAGGGACAGCCTAGGGAGCCACAGGTGTACACCCTGCC
CCCTTCTCAGGAGGAGATGACAAAGAACCAGGTGTCCCTGACCTGTCTGGTGAAG
GGCTTCTATCCAAGCGACATCGCTGTGGAGTGGGAGTCTAATGGCCAGCCCGAGA
ACAATTACAAGACCACACCACCCGTGCTGGACTCTGATGGCTCCTTCTTTCTGTAT
TCTAGGCTGACAGTGGATAAGTCCCGGTGGCAGGAGGGCAACGTGTTTAGCTGCT
CTGTGATGCACGAGGCCCTGCACAATCATTATACCCAGAAGTCCCTGAGCCTGTCT
CTGGGCAAG
Amino acid sequence of polypeptide chain H2 (SEQ ID NO: 10) 
EVQLVESGGGLVQPGGSLRLSCAASGYTYSRHCLGWFRQAPGKGREAVSTIDSD
GSTSYADSVKGRFTISRDNAKNTLYLQMNSLRPEDTAVYYCAIGPNPRYCSGAPNTRG
AEHYFGYWGQGTLVTVSSEPKSSDKTHTSPPSPEVQLVQSGAEVKKPGSSVKVSCKA
SGYSFTHHWIHWVRQAPGQGLEWMGMIDASDSETRLSQKFKDRVTITADKSTSTAY
MELSSLRSEDTAVYYCARLGRYYFDYWGQGTTVTVSSASTKGPSVFPLAPCSRSTSES
TAALGCLVKDYFPEPVTVSWNSGALTSGVHTFPAVLQSSGLYSLSSVVTVPSSSLGTK
TYTCNVDHKPSNTKVDKRVESKYGPPCPPCPAPEFEGGPSVFLFPPKPKDTLMISRTPE
VTCVVVDVSQEDPEVQFNWYVDGVEVHNAKTKPREEQFNSTYRVVSVLTVLHQDW
 
LNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVM
HEALHNHYTQKSLSLSLGK
DNA sequence of polypeptide chain H3 (SEQ ID NO: 11) 
GAGGTGCAGCTGGTGCAGTCCGGAGCTGAGGTGAAGAAGCCAGGATCCAGC
GTGAAGGTGAGCTGCAAGGCTAGCGGCTACTCTTTCACCCACCATTGGATCCACT
GGGTGAGGCAGGCTCCTGGACAGGGACTGGAGTGGATGGGCATGATCGACGCTT
CCGATAGCGAGACAAGACTGTCTCAGAAGTTTAAGGACCGCGTGACCATCACAGC
CGATAAGTCTACCTCCACAGCTTACATGGAGCTGTCTTCCCTGAGATCCGAGGACA
CCGCCGTGTACTATTGTGCTAGGCTGGGCCGGTACTATTTCGATTATTGGGGCCAG
GGCACCACAGTGACAGTGAGCTCTGCCAGCACAAAGGGCCCTTCCGTGTTCCCA
CTGGCTCCCTGCTCCAGAAGCACATCTGAGTCCACCGCCGCTCTGGGCTGTCTGG
TGAAGGACTACTTCCCTGAGCCAGTGACCGTGTCCTGGAACAGCGGCGCCCTGAC
ATCTGGCGTGCACACCTTTCCAGCTGTGCTGCAGTCCAGCGGCCTGTACTCCCTGT
CTTCCGTGGTGACAGTGCCCAGCTCTTCCCTGGGCACCAAGACATATACCTGCAA
CGTGGACCATAAGCCTTCCAATACCAAGGTGGATAAGAGGGTGGAGAGCAAGTAC
GGACCACCTTGCCCACCATGTCCAGCTCCTGAGTTTGAGGGAGGACCATCCGTGT
TCCTGTTTCCTCCAAAGCCTAAGGACACCCTGATGATCAGCCGGACACCTGAGGT
GACCTGCGTGGTGGTGGACGTGTCTCAGGAGGATCCAGAGGTGCAGTTCAACTG
GTACGTGGATGGCGTGGAGGTGCACAATGCTAAGACCAAGCCAAGAGAGGAGCA
GTTTAATTCCACATACCGCGTGGTGAGCGTGCTGACCGTGCTGCATCAGGATTGGC
TGAACGGCAAGGAGTATAAGTGCAAGGTGTCCAATAAGGGCCTGCCCAGCTCTAT
CGAGAAGACAATCAGCAAGGCTAAGGGACAGCCTAGGGAGCCACAGGTGTACAC
CCTGCCCCCTTCTCAGGAGGAGATGACAAAGAACCAGGTGTCCCTGACCTGTCTG
GTGAAGGGCTTCTATCCAAGCGACATCGCTGTGGAGTGGGAGTCTAATGGCCAGC
CCGAGAACAATTACAAGACCACACCACCCGTGCTGGACTCTGATGGCTCCTTCTT
TCTGTATTCTAGGCTGACAGTGGATAAGTCCCGGTGGCAGGAGGGCAACGTGTTT
AGCTGCTCTGTGATGCACGAGGCCCTGCACAATCATTATACCCAGAAGTCCCTGAG
CCTGTCTCTGGGCAAGGGTGGAGGCGGTAGTGGAGGCGGTGGTTCAGGCGGAGG
CGGATCTGAGGTGCAGCTGGTGGAATCTGGGGGAGGACTGGTGCAGCCTGGGGG
AAGCCTGAGACTGAGTTGTGCCGCAAGCGGATACACTTACAGCAGGCACTGCCTG
GGATGGTTCCGACAGGCACCAGGCAAGGGACGAGAGGCCGTGTCTACCATCGAC
AGTGATGGGAGCACATCCTACGCTGACTCAGTCAAGGGACGGTTTACTATCAGCA
GAGATAACGCAAAGAACACCCTGTATCTGCAGATGAACTCCCTGAGACCAGAAGA
 
CACAGCCGTGTACTATTGCGCTATCGGACCCAACCCTCGGTACTGTTCTGGCGCCC
CCAATACCCGGGGAGCCGAACATTACTTTGGGTATTGGGGACAGGGCACACTGGT
GACCGTGAGCAGC
Amino acid sequence of polypeptide chain H3 (SEQ ID NO: 12) 
EVQLVQSGAEVKKPGSSVKVSCKASGYSFTHHWIHWVRQAPGQGLEWMGMID
ASDSETRLSQKFKDRVTITADKSTSTAYMELSSLRSEDTAVYYCARLGRYYFDYWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCPP
CPAPEFEGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGKGGGGSGGGG
SGGGGSEVQLVESGGGLVQPGGSLRLSCAASGYTYSRHCLGWFRQAPGKGREAVSTI
DSDGSTSYADSVKGRFTISRDNAKNTLYLQMNSLRPEDTAVYYCAIGPNPRYCSGAPN
TRGAEHYFGYWGQGTLVTVSS
DNA sequence of polypeptide chain H4 (SEQ ID NO: 13) 
GAGGTGCAGCTGGTGGAATCTGGGGGAGGACTGGTGCAGCCTGGGGGAAGC
CTGAGACTGAGTTGTGCCGCAAGCGGATACACTTACAGCAGGCACTGCCTGGGAT
GGTTCCGACAGGCACCAGGCAAGGGACGAGAGGCCGTGTCTACCATCGACAGTG
ATGGGAGCACATCCTACGCTGACTCAGTCAAGGGACGGTTTACTATCAGCAGAGA
TAACGCAAAGAACACCCTGTATCTGCAGATGAACTCCCTGAGACCAGAAGACACA
GCCGTGTACTATTGCGCTATCGGACCCAACCCTCGGTACTGTTCTGGCGCCCCCAA
TACCCGGGGAGCCGAACATTACTTTGGGTATTGGGGACAGGGCACACTGGTGACC
GTGAGCAGCGGTGGAGGCGGTAGTGGAGGCGGTGGTTCAGGCGGAGGCGGATCT
GAGGTGCAGCTGGTGCAGTCCGGAGCTGAGGTGAAGAAGCCAGGATCCAGCGTG
AAGGTGAGCTGCAAGGCTAGCGGCTACTCTTTCACCACCATTGGATCCACTGGG
TGAGGCAGGCTCCTGGACAGGGACTGGAGTGGATGGGCATGATCGACGCTTCCGA
TAGCGAGACAAGACTGTCTCAGAAGTTTAAGGACCGCGTGACCATCACAGCCGAT
AAGTCTACCTCCACAGCTTACATGGAGCTGTCTTCCCTGAGATCCGAGGACACCG
CCGTGTACTATTGTGCTAGGCTGGGCCGGTACTATTTCGATTATTGGGGCCAGGGC
ACCACAGTGACAGTGAGCTCTGCCAGCACAAAGGGCCCTTCCGTGTTCCCACTGG
CTCCCTGCTCCAGAAGCACATCTGAGTCCACCGCCGCTCTGGGCTGTCTGGTGAA
GGACTACTTCCCTGAGCCAGTGACCGTGTCCTGGAACAGCGGCGCCCTGACATCT
GGCGTGCACACCTTTCCAGCTGTGCTGCAGTCCAGCGGCCTGTACTCCCTGTCTTC
 
CGTGGTGACAGTGCCCAGCTCTTCCCTGGGCACCAAGACATATACCTGCAACGTG
GACCATAAGCCTTCCAATACCAAGGTGGATAAGAGGGTGGAGAGCAAGTACGGAC
CACCTTGCCCACCATGTCCAGCTCCTGAGTTTGAGGGAGGACCATCCGTGTTCCT
GTTTCCTCCAAAGCCTAAGGACACCCTGATGATCAGCCGGACACCTGAGGTGACC
TGCGTGGTGGTGGACGTGTCTCAGGAGGATCCAGAGGTGCAGTTCAACTGGTACG
TGGATGGCGTGGAGGTGCACAATGCTAAGACCAAGCCAAGAGAGGAGCAGTTTA
ATTCCACATACCGCGTGGTGAGCGTGCTGACCGTGCTGCATCAGGATTGGCTGAA
CGGCAAGGAGTATAAGTGCAAGGTGTCCAATAAGGGCCTGCCCAGCTCTATCGAG
AAGACAATCAGCAAGGCTAAGGGACAGCCTAGGGAGCCACAGGTGTACACCCTG
CCCCCTTCTCAGGAGGAGATGACAAAGAACCAGGTGTCCCTGACCTGTCTGGTGA
AGGGCTTCTATCCAAGCGACATCGCTGTGGAGTGGGAGTCTAATGGCCAGCCCGA
GAACAATTACAAGACCACACCACCCGTGCTGGACTCTGATGGCTCCTTCTTTCTGT
ATTCTAGGCTGACAGTGGATAAGTCCCGGTGGCAGGAGGGCAACGTGTTTAGCTG
CTCTGTGATGCACGAGGCCCTGCACAATCATTATACCCAGAAGTCCCTGAGCCTGT
CTCTGGGCAAG
Amino acid sequence of polypeptide chain H4 (SEQ ID NO: 14) 
EVQLVESGGGLVQPGGSLRLSCAASGYTYSRHCLGWFRQAPGKGREAVSTIDSD
GSTSYADSVKGRFTISRDNAKNTLYLQMNSLRPEDTAVYYCAIGPNPRYCSGAPNTRG
AEHYFGYWGQGTLVTVSSGGGGSGGGGSGGGGSEVQLVQSGAEVKKPGSSVKVSC
KASGYSFTHHWIHWVRQAPGQGLEWMGMIDASDSETRLSQKFKDRVTITADKSTST
AYMELSSLRSEDTAVYYCARLGRYYFDYWGQGTTVTVSSASTKGPSVFPLAPCSRST
SESTAALGCLVKDYFPEPVTVSWNSGALTSGVHTFPAVLQSSGLYSLSSVVTVPSSSLG
TKTYTCNVDHKPSNTKVDKRVESKYGPPCPPCPAPEFEGGPSVFLFPPKPKDTLMISR
TPEVTCVVVDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTKNQVSLTCL
VKGFYPSDIAVEWESNGQPENNYKTTPPVLDSDGSFFLYSRLTVDKSRWQEGNVFSC
SVMHEALHNHYTQKSLSLSLGK
DNA sequence of polypeptide chain L1 (SEQ ID NO: 15) 
GAGGTGCAGCTGGTGGAATCTGGGGGAGGACTGGTGCAGCCTGGGGGAAGC
CTGAGACTGAGTTGTGCCGCAAGCGGATACACTTACAGCAGGCACTGCCTGGGAT
GGTTCCGACAGGCACCAGGCAAGGGACGAGAGGCCGTGTCTACCATCGACAGTG
ATGGGAGCACATCCTACGCTGACTCAGTCAAGGGACGGTTTACTATCAGCAGAGA
TAACGCAAAGAACACCCTGTATCTGCAGATGAACTCCCTGAGACCAGAAGACACA
GCCGTGTACTATTGCGCTATCGGACCCAACCCTCGGTACTGTTCTGGCGCCCCCAA
 
TACCCGGGGAGCCGAACATTACTTTGGGTATTGGGGACAGGGCACACTGGTGACC
GTGAGCAGCGGTGGAGGCGGTAGTGGAGGCGGTGGTTCAGGCGGAGGCGGATCT
GAGATCGTGCTGACCCAGTCTCCAGCCACACTGTCTCTGTCCCCAGGAGAGAGG
CCACCCTGAGCTGCCGGGCTTCTGAGAACGTGGGCACATACATCTCCTGGTATCA
GCAGAAGCCAGGACAGGCTCCTAGGCTGCTGATCTACGGCGCTAGCAATAGATAT
ACCGGCATCCCTGCTCGCTTCAGCGGATCTGGATCCGGCACAGACTTTACCCTGAC
AATCTCCAGCCTGGAGCCAGAGGATTTCGCCGTGTACTATTGTGGCGAGTCCTACG
GCCACCTGTATACCTTTGGCGGCGGCACAAAGGTGGAGATCAAGCGAACGGTGGC
TGCACCATCTGTCTTCATCTTCCCGCCATCTGATGAGCAGTTGAAATCTGGAACTG
CCTCTGTTGTGTGCCTGCTGAATAACTTCTATCCCAGAGAGGCCAAAGTACAGTGG
AAGGTGGATAACGCCCTCCAATCGGGTAACTCCCAGGAGAGTGTCACAGAGCAG
GACAGCAAGGACAGCACCTACAGCCTCAGCAGCACCCTGACGCTGAGCAAAGCA
GACTACGAGAAACACAAAGTCTACGCCTGCGAAGTCACCCATCAGGGCCTGAGC
TCGCCCGTCACAAAGAGCTTCAACAGGGGAGAGTGT
Amino acid sequence of polypeptide chain L1 (SEQ ID NO: 16) 
EVQLVESGGGLVQPGGSLRLSCAASGYTYSRHCLGWFRQAPGKGREAVSTIDSD
GSTSYADSVKGRFTISRDNAKNTLYLQMNSLRPEDTAVYYCAIGPNPRYCSGAPNTRG
AEHYFGYWGQGTLVTVSSGGGGSGGGGSGGGGSEIVLTQSPATLSLSPGERATLSCR
ASENVGTYISWYQQKPGQAPRLLIYGASNRYTGIPARFSGSGSGTDFTLTISSLEPEDF
AVYYCGESYGHLYTFGGGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVT
HQGLSSPVTKSFNRGEC
DNA sequence of polypeptide chain L2 (SEQ ID NO: 17) 
GAGGTGCAGCTGGTGGAATCTGGGGGAGGACTGGTGCAGCCTGGGGGAAGC
CTGAGACTGAGTTGTGCCGCAAGCGGATACACTTACAGCAGGCACTGCCTGGGAT
GGTTCCGACAGGCACCAGGCAAGGGACGAGAGGCCGTGTCTACCATCGACAGTG
ATGGGAGCACATCCTACGCTGACTCAGTCAAGGGACGGTTTACTATCAGCAGAGA
TAACGCAAAGAACACCCTGTATCTGCAGATGAACTCCCTGAGACCAGAAGACACA
GCCGTGTACTATTGCGCTATCGGACCCAACCCTCGGTACTGTTCTGGCGCCCCCAA
TACCCGGGGAGCCGAACATTACTTTGGGTATTGGGGACAGGGCACACTGGTGACC
GTGAGCAGCGGTGGAGGCGGTAGTGGAGGCGGTTCAGAGATCGTGCTGACCCAG
TCTCCAGCCACACTGTCTCTGTCCCCAGGAGAGAGGGCCACCCTGAGCTGCCGGG
CTTCTGAGAACGTGGGCACATACATCTCCTGGTATCAGCAGAAGCCAGGACAGGC
TCCTAGGCTGCTGATCTACGGCGCTAGCAATAGATATACCGGCATCCCTGCTCGCTT
 
CAGCGGATCTGGATCCGGCACAGACTTTACCCTGACAATCTCCAGCCTGGAGCCA
GAGGATTTCGCCGTGTACTATTGTGGCGAGTCCTACGGCCACCTGTATACCTTTGG
CGGCGGCACAAAGGTGGAGATCAAGCGAACGGTGGCTGCACCATCTGTCTTCATC
TTCCCGCCATCTGATGAGCAGTTGAAATCTGGAACTGCCTCTGTTGTGTGCCTGCT
GAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACC
TACAGCCTCAGCAGCACCCTGACGCTGAGCAAAGCAGACTACGAGAAACACAAA
GTCTACGCCTGCGAAGTCACCCATCAGGGCCTGAGCTCGCCCGTCACAAAGAGCT
TCAACAGGGGAGAGTGT
Amino acid sequence of polypeptide chain L2 (SEQ ID NO: 18) 
EVQLVESGGGLVQPGGSLRLSCAASGYTYSRHCLGWFRQAPGKGREAVSTIDSD
GSTSYADSVKGRFTISRDNAKNTLYLQMNSLRPEDTAVYYCAIGPNPRYCSGAPNTRG
AEHYFGYWGQGTLVTVSSGGGGSGGGSEIVLTQSPATLSLSPGERATLSCRASENVGT
YISWYQQKPGQAPRLLIYGASNRYTGIPARFSGSGSGTDFTLTISSLEPEDFAVYYCGE
SYGHLYTFGGGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPV
TKSFNRGEC
DNA sequence of polypeptide chain H5 (SEQ ID NO: 19) 
GAGGTGCAGCTGGTGGAATCTGGGGGAGGACTGGTGCAGCCTGGGGGAAGC
CTGAGACTGAGTTGTGCCGCAAGCGGATACACTTACAGCAGGCACTGCCTGGGAT
GGTTCCGACAGGCACCAGGCAAGGACGAGAGGCCGTGTCTACCATCGACAGTG
ATGGGAGCACATCCTACGCTGACTCAGTCAAGGGACGGTTTACTATCAGCAGAGA
TAACGCAAAGAACACCCTGTATCTGCAGATGAACTCCCTGAGACCAGAAGACACA
GCCGTGTACTATTGCGCTATCGGACCCAACCCTCGGTACTGTTCTGGCGCCCCCAA
TACCCGGGGAGCCGAACATTACTTTGGGTATTGGGGACAGGGCACACTGGTGACC
GTGAGCAGCGAGAGCAAGTACGGACCACCTTGCCCACCATGTCCAGCTCCTGAGT
TTGAGGGAGGACCATCCGTGTTCCTGTTTCCTCCAAAGCCTAAGGACACCCTGAT
GATCAGCCGGACACCTGAGGTGACCTGCGTGGTGGTGGACGTGTCTCAGGAGGAT
CCAGAGGTGCAGTTCAACTGGTACGTGGATGGCGTGGAGGTGCACAATGCTAAGA
CCAAGCCAAGAGAGGAGCAGTTTAATTCCACATACCGCGTGGTGAGCGTGCTGAC
CGTGCTGCATCAGGATTGGCTGAACGGCAAGGAGTATAAGTGCAAGGTGTCCAAT
AAGGGCCTGCCCAGCTCTATCGAGAAGACAATCAGCAAGGCTAAGGGACAGCCT
AGGGAGCCACAGGTGTACACCCTGCCCCCTTCTCAGGAGGAGATGACAAAGAAC
CAGGTGTCCCTGACCTGTCTGGTGAAGGGCTTCTATCCAAGCGACATCGCTGTGG
 
AGTGGGAGTCTAATGGCCAGCCCGAGAACAATTACAAGACCACACCACCCGTGCT
GGACTCTGATGGCTCCTTCTTTCTGTATTCTAGGCTGACAGTGGATAAGTCCCGGT
GGCAGGAGGGCAACGTGTTTAGCTGCTCTGTGATGCACGAGGCCCTGCACAATCA
TTATACCCAGAAGTCCCTGAGCCTGTCTCTGGGCAAG
Amino acid sequence of polypeptide chain H5 (SEQ ID NO: 20)

EVQLVESGGGLVQPGGSLRLSCAASGYTYSRHCLGWFRQAPGKGREAVSTIDSD
GSTSYADSVKGRFTISRDNAKNTLYLQMNSLRPEDTAVYYCAIGPNPRYCSGAPNTRG
AEHYFGYWGQGTLVTVSSESKYGPPCPPCPAPEFEGGPSVFLFPPKPKDTLMISRTPEV
TCVVVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCSVMH
EALHNHYTQKSLSLSLGK

**Table 4. Linker sequences and IgG4-Fc sequences**

| | **Sequence** | **SEQ ID NO:** |
|---|---|---|
| **DNA sequence of E-Linker** | GAACCTAAGTCTAGCGACAAAACTCATACCAGCCCCCC TAGTCCA | 32 |
| **Amino acid sequence of E-Linker** | EPKSSDKTHTSPPSP | 33 |
| **DNA sequence of G15-Linker** | GGTGGAGGCGGTAGTGGAGGCGGTGGTTCAGGCGGAG GCGGATCT | 34 |
| **Amino acid sequence of G15-Linker** | GGGGSGGGGSGGGGS | 35 |
| **DNA sequence of G9-Linker** | GGTGGAGGCGGTAGTGGAGGCGGTTCA | 36 |
| **Amino acid sequence of G9- Linker** | GGGGSGGGS | 37 |
| **DNA sequence of IgG4 Fc** | GAGAGCAAGTACGGACCACCTTGCCCACCATGTCCAGC TCCTGAGTTTGAGGGAGGACCATCCGTGTTCCTGTTTCC TCCAAAGCCTAAGGACACCCTGATGATCAGCCGGACAC CTGAGGTGACCTGCGTGGTGGTGGACGTGTCTCAGGAG GATCCAGAGGTGCAGTTCAACTGGTACGTGGATGGCGT GGAGGTGCACAATGCTAAGACCAAGCCAAGAGAGGAG CAGTTTAATTCCACATACCGCGTGGTGAGCGTGCTGACC GTGCTGCATCAGGATTGGCTGAACGGCAAGGAGTATAA GTGCAAGGTGTCCAATAAGGGCCTGCCCAGCTCTATCG AGAAGACAATCAGCAAGGCTAAGGGACAGCCTAGGGA | 38 |
| | GCCACAGGTGTACACCCTGCCCCCTTCTCAGGAGGAGA TGACAAAGAACCAGGTGTCCCTGACCTGTCTGGTGAAG GGCTTCTATCCAAGCGACATCGCTGTGGAGTGGGAGTC TAATGGCCAGCCCGAGAACAATTACAAGACCACACCAC CCGTGCTGGACTCTGATGGCTCCTTCTTTCTGTATTCTAG GCTGACAGTGGATAAGTCCCGGTGGCAGGAGGGCAAC GTGTTTAGCTGCTCTGTGATGCACGAGGCCCTGCACAAT CATTATACCCAGAAGTCCCTGAGCCTGTCTCTGGGCAAG | |
| **Amino acid sequence of IgG4 Fc** | ESKYGPPCPPCPAPEFEGGPSVFLFPPKPKDTLMISRTPEVT CVVVDVSQEDPEVQFNWYVDGVEVHNAKTKPREEQFNS TYRVVSVLTVLHQDWLNGKEYKCKVSNKGLPSSIEKTISK AKGQPREPQVYTLPPSQEEMTKNQVSLTCLVKGFYPSDIA VEWESNGQPENNYKTTPPVLDSDGSFFLYSRLTVDKSRW QEGNVFSCSVMHEALHNHYTQKSLSLSLGK | 39 |

### Example 2 FACS affinity analysis

For the constructed series of bispecific antibodies, the affinity of these samples with antigens was measured by using a flow cytometer. A sample with an initial concentration of 300 nM undergone serial dilution by 3 times, and then the affinities between samples with different concentrations and CTLA-4 antigens or CD47 antigens expressed on CHO-K1 cells were separately tested. Then, an antibody-antigen binding curve was generated based on the geometric mean, raw data of four parameters was plotted by using the GRAPHPAD Prism V6.02 software, and a best fitted value program was compiled to analyze EC₅₀.

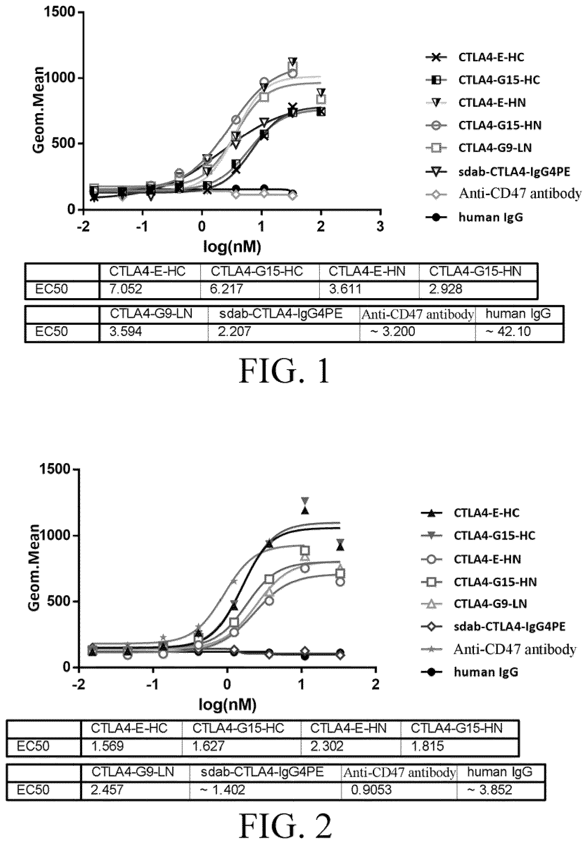

For the affinity analysis of the CTLA-4 antigen, after the bispecific antibody produced when the CTLA-4 sdAb was fused to the N-terminus or C-terminus of the heavy chain or light chain of the anti-CD47 mAb was incubated on the CHO-K1 cells expressing the CTLA-4 antigen, it was found through FACS detection that, considering the EC₅₀ value and the Y-axis response value, compared with a control of the CTLA-4 sdAb fused to IgG4 Fc (sdAb-CTLA4-IgG4PE), the affinity between the CTLA-4 antigen and the bispecific antibody that was produced when the CTLA-4 sdAb was fused to the N-terminus of the heavy chain or light chain of the anti-CD47 mAb was higher than that of the sdAb control. The affinity between the CTLA-4 antigen and the bispecific antibody that was produced when the CTLA-4 sdAb was fused to the C-terminus of the heavy chain of the anti-CD47 mAb was lower than that of the sdAb control (shown in FIG. 1). Therefore, linking the CTLA-4 sdAb to the N-terminus of the CD47 mAb may enhance the binding of the CTLA-4 sdAb to the CTLA-4 antigen, and linking the CTLA-4 sdAb to the C-terminus of the CD47 mAb reduces the affinity between the CTLA-4 sdAb and the CTLA-4 antigen.

For the affinity analysis of the CD47 antigen, after the bispecific antibody produced when the CTLA-4 sdAb was fused to the terminus of the heavy chain or light chain of the anti-CD47 mAb was incubated on the CHO-K1 cells expressing the CD47 antigen, it was found through FACS detection that, compared with a control of the anti-CD47 mAb, the affinity between the CD47 antigen and the bispecific antibody that was produced when the CTLA-4 sdAb was fused to the C-terminus of the heavy chain of the anti-CD47 mAb was close to the affinity between the CD47 antigen and the control of the anti-CD47 mAb. The affinity between the CD47 antigen and the bispecific antibody that was produced when the CTLA-4 sdAb was fused to the N-terminus of the heavy chain or light chain of the anti-CD47 mAb was lower than the affinity between the CD47 antigen and the control of the anti-CD47 mAb (shown in FIG. 2). Therefore, linking the CTLA-4 sdAb to the C-terminus of the anti-CD47 mAb maintains the binding of the anti-CD47 antibody to the CD47 antigen, and linking the CTLA-4 sdAb to the N-terminus of the anti-CD47 mAb reduces the affinity between the CD47 antibody and the CD47 antigen.

### Example 3 Biological activity measurement in vitro

For the biological activity measurement *in vitro* of the anti-CD47/anti-CTLA-4 bispecific antibody, there is no analytical system that can detect both CD47 and CTLA-4 blockers at the same time. Therefore, the bioassay of the CTLA-4 blocker is carried out by using the Promega test kit, and then the activity of the bispecific antibody is tested through a cell phagocytosis experiment of the anti-CD47 antibody.

The Promega CTLA-4 blocking function reporter gene kit (CTLA4 Blockade Bioassay, Promega kit product number JA3001) is used in the experiment for testing the in vitro function of anti-CTLA-4 mAb samples. The kit detection system consists of two genetically engineered cell lines. The stimulating cell line is aAPC/Raji cells, which express an engineered cell surface protein (the protein can activate the homologous TCR in an antigen-independent manner), and endogenously express the CTLA-4 ligands CD80 and CD86. The effector cell line is the Jurkat T cell line, which stably expresses human CTLA4 and a luciferase reporter gene driven by a natural promoter that responds to TCR/CD28 activation. When the two types of cells are co-cultured, CTLA-4 competes with CD28 for their shared ligands CD80 and CD86, and therefore inhibits CD28 pathway activation and promoter-mediated luminescence. The addition of the anti-CTLA-4 antibody can block the interaction between CTLA-4 and its ligands CD80 and CD86, thereby restoring promoter-mediated chemiluminescence.

The effector cell line Jurkat T cells were first plated in a 96-well plate, and the anti-CD47/anti-CTLA-4 bispecific antibody and the stimulating cell line aAPC/Raji cells were then added. The resulting system was incubated at 37°C for 6 h. Next, the Bio-Glo^{™} fluorescence detection reagent was added to the system, and then incubated for 5-10 min at room temperature. Finally, fluorescence signals in the 96-well plate were read by using a chemical fluorescence signal plate reader. The experiment used the form of eight concentrations and triplicated wells, used the relative fluorescence value as the y-axis, and used the concentration of antibody samples as the x-axis, to plot a four-parameter curve. The curve was analyzed by using the GraphPad Prism software to obtain the EC₅₀ value of the anti-CD47/anti-CTLA-4 bispecific antibody sample.

For the cell phagocytosis experiment of the anti-CD47 antibody, PBMCs were first extracted from human peripheral blood by the concentration gradient method. Monocytes were then isolated from the PBMCs by using the whole monocyte isolation kit (Miltenyi Biotech). These monocytes were stimulated into macrophages with GM-CSF within 14 days. On day 14, HL60 cells were stained with the PKH26 dye and then seeded in a 96-well culture plate, MDM was digested from a Petri dish by using Accutase and then added into the culture plate in which HL60 cells stained with PKH26 were seeded, then the anti-CD47/anti-CTLA-4 bispecific antibody sample after serial dilution was added, and incubated at 37°C for 1 h to carry out the cell phagocytosis reaction. One hour later, MDM was digested from the cell culture plate and stained with the fluorescently labeled anti-CD 11b antibody. The cells in the cell culture plate were then analyzed by using the BD FACSCalibur flow cytometer. The phagocytic percentage was calculated by dividing the number of PKH26 and CD11b double-positive cells by the number of PKH26 single-positive cells. A dose-response curve used the phagocytic percentage as the y-axis and used the concentration of antibody samples as the x-axis, and the GraphPad Prism software was used for analysis to obtain the EC₅₀ value and other curve parameters.

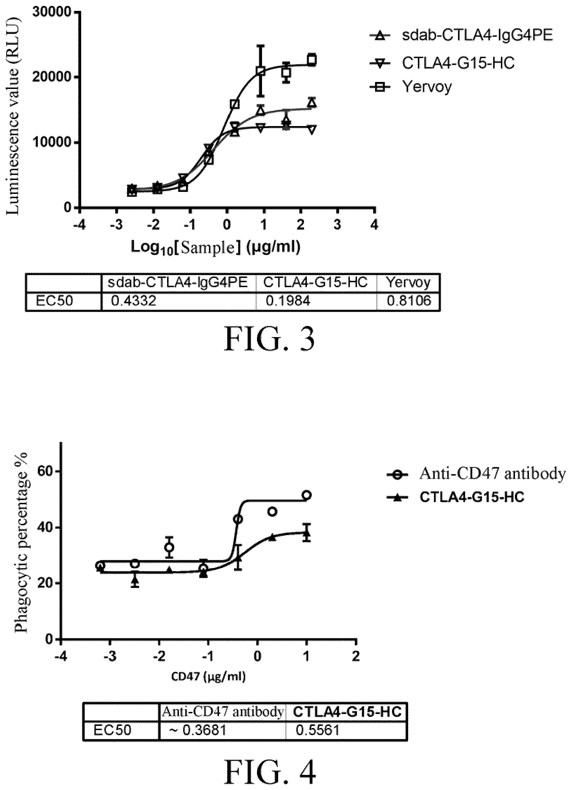

It was indicated based on the biological activity measurement result of the CTLA-4 blocker that the biological activity of the bispecific antibody CTLA4-G15-HC was lower than that of the CTLA-4 sdAb control (sdAb-CTLA4-IgG4PE), and was also lower than that of the CTLA-4 mAb control (Yervoy) (shown in FIG. 3). The reduction of the CTLA-4 blocking activity in the bispecific antibody CTLA4-G15-HC may reduce or alleviate the clinical side effects caused by the high-affinity CTLA-4 blocking antibody to a certain extent.

It was indicated based on the cell phagocytosis experiment result of the anti-CD47 antibody that the EC₅₀ value of the bispecific antibody CTLA4-G15-HC was slightly lower than that of the CD47 antibody control (shown in FIG. 4). This may also reduce the clinical red blood cell toxicity caused by binding to normal red blood cells to a certain extent.

## Claims

1. An isolated anti-CD47/anti-CTLA-4 bispecific antigen-binding protein or a fragment thereof, comprising (a) a first antigen binding portion comprising a heavy chain variable region (V_{H}) and a light chain variable region (V_{L}), wherein the V_{H} and V_{L} form an antigen binding site that specifically binds to CD47; and (b) a second antigen binding portion comprising a single-domain antibody (sdAb) that specifically binds to CTLA-4, wherein the first antigen binding portion and the second antigen binding portion are fused to each other.

2. The bispecific antigen-binding protein or the fragment thereof according to claim 1, wherein the V_{H} of the first antigen binding portion comprises heavy chain complementarity-determining regions (CDRs) HCDR1, HCDR2, and HCDR3, the amino acid sequences of the HCDR1, HCDR2, and HCDR3 are respectively as set forth in SEQ ID NO:21, SEQ ID NO:22, and SEQ ID NO:23, or the sequences respectively comprising at most three amino acid mutations thereto; and the V_{L} of the first antigen binding portion comprises light chain CDRs LCDR1, LCDR2, and LCDR3, the amino acid sequences of the LCDR1, LCDR2, and LCDR3 are respectively as set forth in SEQ ID NO:24, SEQ ID NO:25, and SEQ ID NO:26, or the sequences respectively comprising at most three amino acid mutations thereto.

3. The bispecific antigen-binding protein or the fragment thereof according to claim 1, wherein the sdAb of the second antigen binding portion comprises CDRs CDR1, CDR2, and CDR3, the amino acid sequences of the CDRs CDR1, CDR2, and CDR3 are respectively as set forth in SEQ ID NO:29, SEQ ID NO:30, and SEQ ID NO:31, or the sequences respectively comprising at most three amino acid mutations thereto.

4. The bispecific antigen-binding protein or the fragment thereof according to claim 1, wherein the first antigen binding portion is a full-length antibody comprising two heavy chains and two light chains, the heavy chain comprises V_{H}, and the light chain comprises V_{L}.

5. The bispecific antigen-binding protein or the fragment thereof according to any one of claims 1 to 4, wherein the C-terminus of the second antigen binding portion is fused to the N-terminus of at least one heavy chain of the first antigen binding portion or the N-terminus of at least one light chain of the first antigen binding portion.

6. The bispecific antigen-binding protein or the fragment thereof according to any one of claims 1 to 4, wherein the N-terminus of the second antigen binding portion is fused to the C-terminus of at least one heavy chain of the first antigen binding portion or the C-terminus of at least one light chain of the first antigen binding portion.

7. The bispecific antigen-binding protein or the fragment thereof according to any one of claims 1 to 6, wherein the first antigen binding portion and the second antigen binding portion are fused by a peptide bond or a peptide linker.

8. The bispecific antigen-binding protein or the fragment thereof according to claim 7, wherein the peptide linker is selected from a mutated human IgG1 hinge region or a GS linker, preferably, an amino acid sequence of the peptide linker is as set forth in SEQ ID NO:33, SEQ ID NO:35, or SEQ ID NO:37.

9. The bispecific antigen-binding protein or the fragment thereof according to any one of claims 1 to 8, wherein the heavy chain of the first antigen binding portion comprises a sequence that is at least 95% identical to the amino acid sequence as set forth in SEQ ID NO:4, and the light chain of the first antigen binding portion comprises a sequence that is at least 95% identical to the amino acid sequence as set forth in SEQ ID NO:6.

10. The bispecific antigen-binding protein or the fragment thereof according to any one of claims 1 to 9, wherein the second antigen binding portion comprises a sequence that is at least 95% identical to the amino acid sequence as set forth in SEQ ID NO:28.

11. The bispecific antigen-binding protein or the fragment thereof according to any one of claims 1 to 10, wherein the first antigen binding portion comprises a human, humanized, or chimeric antibody or a fragment thereof, and the sdAb of the second antigen binding portion is a camelid, chimeric, humanized, or human antibody.

12. The bispecific antigen-binding protein or the fragment thereof according to any one of claims 1 to 11, comprising an anti-CD47 antibody and an anti-CTLA-4 sdAb, with the N-terminus of the anti-CTLA-4 sdAb fused to the C-terminus of two heavy chains of the anti-CD47 antibody, wherein the heavy chain fusion polypeptide comprises a sequence that is at least 95% identical to the amino acid sequence as set forth in SEQ ID NO:8 or SEQ ID NO: 12, and the light chain polypeptide comprises a sequence that is at least 95% identical to the amino acid sequence as set forth in SEQ ID NO:6.

13. The bispecific antigen-binding protein or the fragment thereof according to any one of claims 1 to 11, comprising an anti-CD47 antibody and an anti-CTLA-4 sdAb, with the C-terminus of the anti-CTLA-4 sdAb fused to the N-terminus of two heavy chains of the anti-CD47 antibody, wherein the heavy chain fusion polypeptide comprises a sequence that is at least 95% identical to the amino acid sequence as set forth in SEQ ID NO: 10 or SEQ ID NO: 14, and the light chain polypeptide comprises a sequence that is at least 95% identical to the amino acid sequence as set forth in SEQ ID NO:6.

14. The bispecific antigen-binding protein or the fragment thereof according to any one of claims 1 to 11, comprising an anti-CD47 antibody and an anti-CTLA-4 sdAb, with the C-terminus of the anti-CTLA-4 sdAb fused to the N-terminus of two light chains of the anti-CD47 antibody, wherein the light chain fusion polypeptide comprises a sequence that is at least 95% identical to the amino acid sequence as set forth in SEQ ID NO: 16 or SEQ ID NO: 18, and the heavy chain polypeptide comprises a sequence that is at least 95% identical to the amino acid sequence as set forth in SEQ ID NO:4.

15. An isolated polynucleotide encoding the anti-CD47/anti-CTLA-4 bispecific antigen-binding protein or the fragment thereof according to any one of claims 1 to 14.

16. A vector comprising the isolated polynucleotide according to claim 15.

17. A host cell comprising the isolated polynucleotide according to claim 15 or the vector according to claim 16.

18. A method for producing an isolated anti-CD47/anti-CTLA-4 bispecific antigen-binding protein or a fragment thereof, comprising culturing the host cell according to claim 17 under proper conditions, and recovering an antibody or a fragment thereof from the cell or a cell culture medium.

19. A pharmaceutical composition, comprising the bispecific antigen-binding protein or the fragment thereof according to any one of claims 1 to 14 and a pharmaceutically acceptable carrier.

20. Use of the anti-CD47/anti-CTLA-4 bispecific antigen-binding protein or the fragment thereof according to any one of claims 1 to 14, the polynucleotide according to claim 15, the vector according to claim 16, and the host cell according to claim 17 in preparing medicines for diseases related to abnormal expression of CD47 and/or CTLA-4.

21. The use according to claim 20, wherein the diseases related to CD47 and/or CTLA-4 are cancers, preferably, the cancers are solid tumors.
